# EUROPEAN PATENT APPLICATION

(11) **EP 4 837 629 A1**
(43) Date of publication of application: **11.11.2026**
(21) Application number: 25736430.7
(22) Date of filing: 05.01.2025
(51) Int. Cl.: A61N 7/02

(54) **FOCUSED ULTRASOUND ABLATION CATHETER AND DEVICE**

(30) Priority: 05.01.2024 CN 202410021541
(71) Applicant: Brattea Medtech Co., Ltd., Shanghai 201114 (CN)
(72) Inventor: GUO, Jiulin, Shanghai 200231 (CN); WANG, Yan, Shanghai 200231 (CN); CHEN, Hong, Shanghai 200231 (CN)
(74) Representative: Wang, Bo
(86) International application number: PCT/CN2025/070630
(87) International publication number: WO 2025/146191

(57) **Abstract**

Disclosed in the present invention are a focused ultrasound ablation catheter and a device. The focused ultrasound ablation catheter comprises a multi-chamber tube as a catheter body; a first ultrasound transducer, arranged at a distal end of the catheter body and electrically connected to a control part, the first ultrasound transducer being parallel to a central axis of the catheter body to be used for measuring a distance and a temperature of an inner wall of a blood vessel, thereby calculating an ablation target region and measuring a temperature of the target region for calculation of an output power of second ultrasound transducers and determination of an ablation end point; and at least two second ultrasound transducers, arranged at the distal end of the catheter body and electrically connected to the control part, the second ultrasound transducers and the central axis of the catheter body forming a set included angle, and the at least two second ultrasound transducers being symmetrical about the first ultrasound transducer for determining an ablation focus and performing focused ablation. By means of the catheter, energy transmitted by sound waves can be concentrated in a focused ablation region, and other regions are not ablated.

## Description

### BACKGROUND

### Technical Field

The present invention belongs to the technical field of medical instruments, and relates to a focused ultrasound ablation catheter, and also a focused ultrasound ablation device including the catheter.

### Related Art

In an ultrasonic ablation technology, using a characteristic that ultrasonic waves can penetrate human body tissue and be focused on a specific region, a transducer inside a balloon is used to emit ultrasonic waves. These ultrasonic waves act on blood vessels and surrounding tissues after passing through physiological saline, and converge energy in a specific target area, thereby achieving sufficient intensity and temperature. As the temperature gradually rises, nerves in and around the blood vessels are ablated, resulting in histopathologically coagulative necrosis of the target area, thereby achieving therapeutic effects on the lesion region while sparing the surrounding normal tissue. However, an existing ultrasonic ablation apparatus cannot implement precise focal ablation. Non-focused ultrasonic energy spreads in a fan-shaped pattern, easily causing damage to the vascular intima and non-target areas, leading to vascular atrophy, stenosis, and deformation, potentially placing the vessels in an occluded state, resulting in adverse reactions in patients and compromising the ablation efficacy.

### SUMMARY

A primary technical problem to be resolved in the present invention is to provide a focused ultrasound ablation catheter.

Another technical problem to be resolved in the present invention is to provide a focused ultrasound ablation device.

To achieve the foregoing technical objectives, the present invention adopts the following technical solutions.

According to a first aspect of embodiments of the present invention, a focused ultrasound ablation catheter is provided, including:
a catheter body, where the catheter body is a multi-lumen tube, and includes at least a guide wire lumen for passage of a guide wire therethrough and a conductor wire lumen for passage of a conductor wire therethrough;
a first ultrasonic transducer, arranged at a distal end of the catheter body, and configured to be electrically connected to a control unit through the conductor wire, where the first ultrasonic transducer is arranged parallel to a central axis of the catheter body and is configured to measure a distance to an inner wall of a blood vessel, to calculate an ablation target area; and the first ultrasonic transducer is further configured to measure a temperature of a target area, which is used for calculating an output power of a second ultrasonic transducer and determining an ablation endpoint; and
at least two second ultrasonic transducers, arranged at the distal end of the catheter body, and configured to be electrically connected to the control unit through the conductor wire, where a set included angle is formed between the second ultrasonic transducer and the central axis of the catheter body, and the at least two second ultrasonic transducers are mutually symmetrical about the first ultrasonic transducer to determine an ablation focal point and perform focused ablation.

Preferably, the second ultrasonic transducer is rotatably arranged at the distal end of the catheter body to adjust a magnitude of the set included angle.

Preferably, centered on the first ultrasonic transducer, the at least two second ultrasonic transducers are movable relative to each other along the central axis of the catheter body, to adjust a relative distance between the at least two ultrasonic transducers.

Preferably, a plurality of first ultrasonic transducers are arranged, and the plurality of first ultrasonic transducers define a circular ring along a circumferential direction of the catheter body; and two second ultrasonic transducers that are obliquely arranged are symmetrically distributed on two sides of each of the first ultrasonic transducers.

Preferably, the second ultrasonic transducer is electrically connected to the controller to receive a current temperature of the ablation target area that is measured by the controller based on the first ultrasonic transducer and calculate a current output power; and
the second ultrasonic transducer emits an ultrasonic wave of a corresponding intensity based on the current output power to perform focused ablation on the ablation target area.

Preferably, the focused ultrasound ablation catheter further includes:
a tip balloon, arranged on an end surface of the distal end of the catheter body, in communication with internal lumens of the catheter body, and configured to occlude a blood vessel in an inflated state; and
the internal lumens include an inflow lumen for fluid infusion and an outflow lumen for fluid drainage.

Preferably, a flexible electrode is mounted on an outer side of the tip balloon, a pressure sensor is mounted on an inner side of the tip balloon, the flexible electrode and the pressure sensor are both electrically connected to the control unit to detect impedance and a water pressure.

According to a second aspect of embodiments of the present invention, a focused ultrasound ablation device is provided, including:
a control unit, configured for automatic control of ultrasonic ablation;
an operating handle, connected to the control unit; and
the focused ultrasound ablation catheter, where a proximal end of the focused ultrasound ablation catheter is mounted to the operating handle and is electrically connected to the control unit; and
the control unit continuously adjusts an output power of the second ultrasonic transducer based on a temperature measured by the first ultrasonic transducer until an ablation endpoint is reached; and the control unit is further configured to determine, based on determination logic, whether the ablation endpoint is reached.

Preferably, the determination logic specifically includes:
when a current temperature of an ablation target area measured by the first ultrasonic transducer reaches a set value and remains stable for a preset duration, and a distance to an inner wall of a blood vessel measured by the first ultrasonic transducer reaches a theoretical value, it is determined that the ablation endpoint is reached.

Preferably, the control unit includes at least:
a main control module, configured to process a signal and data and control automated ablation;
a signal generator, connected to the main control module, and configured to receive and send an ultrasonic signal;
a power amplifier, connected to the signal generator, and configured to amplify the ultrasonic signal;
a gating module, connected to the power amplifier, and configured to select a number of signal channels;
a phase voltage and current module, connected to the main control module, and configured to detect a parameter and output a matching ultrasonic frequency; and
an impedance matching module, connected to the main control module and configured to detect matching impedance and output an ultrasonic signal at a matching power.

Compared with the prior art, the present invention has the following beneficial effects:
1. The distance to the inner wall of the blood vessel is measured through the first ultrasonic transducers that are horizontally arranged, the ablation target area is determined through calculation, and the temperature of the target area is measured. In addition, focusing is performed through the second ultrasonic transducers that are obliquely arranged, so that energy transmitted by acoustic waves is focused on a focal ablation region, and other regions are not ablated, thereby effectively protecting vascular intima.
2. The second ultrasonic transducers are rotatable, so that different focal lengths can be implemented through adjustments, and then an adaptability adjustment can be performed based on different ablation positions for a blood vessel to improve applicability of the focused ultrasound ablation catheter.
3. The second ultrasonic transducers are movable, so that a size of a focused ablation region can be implemented through adjustments, and then an adaptability adjustment can be performed based on different ablation positions for a blood vessel to improve the applicability of the focused ultrasound ablation catheter.
4. The plurality of first ultrasonic transducers and the plurality of second ultrasonic transducers may form a plurality of ablation groups. Any one or several of the ablation groups may be controlled to enter a working state through the control unit, thereby implementing all-around ablation within 360° without rotating a catheter body. This not only improves ablation efficiency, but also improves convenience of an ablation operation.

### BRIEF DESCRIPTION OF THE DRAWINGS

FIG. 1 is a schematic structural diagram of a focused ultrasound ablation catheter according to a first embodiment of the present invention;
FIG. 2 is a cross-sectional view of a catheter body according to the first embodiment of the present invention;
FIG. 3 is a schematic diagram of focused ablation according to the first embodiment of the present invention;
FIG. 4A is a schematic structural diagram of a focused ultrasound ablation catheter according to a second embodiment of the present invention;
FIG. 4B is a schematic structural diagram of a focused ultrasound ablation catheter according to a third embodiment of the present invention;
FIG. 5 is a schematic structural diagram of a focused ultrasound ablation catheter according to a fourth embodiment of the present invention;
FIG. 6 is a schematic structural diagram of a focused ultrasound ablation catheter according to a fifth embodiment of the present invention;
FIG. 7 is a schematic structural diagram of a focused ultrasound ablation device according to a sixth embodiment of the present invention; and
FIG. 8 is a schematic structural diagram of a control unit according to a sixth embodiment of the present invention.

### DETAILED DESCRIPTION

Technical content of the present invention is described in detail below with reference to the accompanying drawings and specific embodiments.

### First embodiment

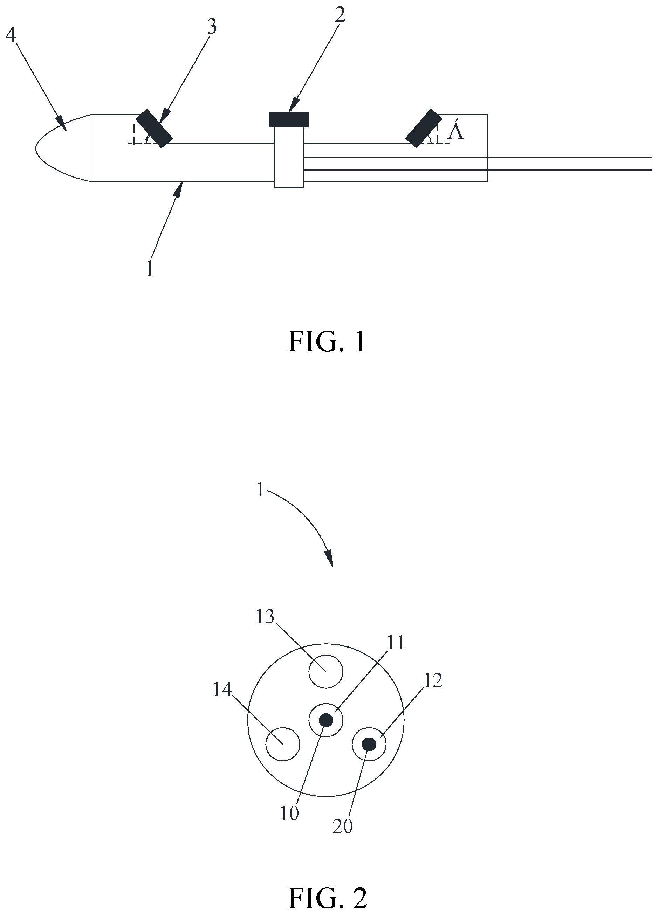

As shown in FIG. 1, the first embodiment of the present invention provides a focused ultrasound ablation catheter including a catheter body 1, a first ultrasonic transducer 2, at least two second ultrasonic transducers 3, and a tip balloon 4. The catheter body 1 is a multi-lumen tube, and lumens respectively have different functions, for example, a guide wire lumen for a guide wire to pass through, a conductor wire lumen for a conductor wire to pass through, and an inflow lumen and an outflow lumen for liquid circulation. In addition, as the position shown in FIG. 1, the first ultrasonic transducer 2 is horizontally arranged and configured to measure a distance to an inner wall of a blood vessel and a temperature thereof, so as to calculate an ablation target area, and measure a current temperature of the ablation target area. The at least two ultrasonic transducers 3 are obliquely arranged and are configured to determine an ablation focal point and perform focused ablation. The tip balloon 4 is arranged at a distal end of the catheter body 1 and configured to occlude a blood vessel in an inflated state.

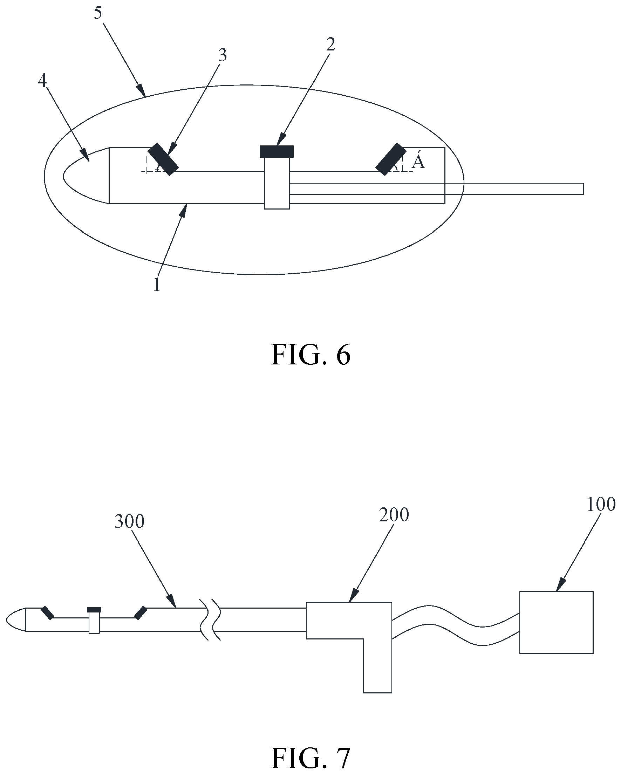

In this embodiment, a proximal end of the catheter body 1 is configured to be connected to a control unit 100 (referring to FIG. 7), and the distal end of the catheter body 1 is configured to extend into a blood vessel. As shown in FIG. 2, the catheter body 1 is a multi-lumen tube, including at least a guide wire lumen 11, a conductor wire lumen 12, an inflow lumen 13, and an outflow lumen 14. A guide wire 10 is arranged in the guide wire lumen 11 to control a bending direction of the catheter body 1 through the guide wire 10 to drive the catheter body 1 to enter different blood vessel tissues. A conductor wire 20 is arranged in the conductor wire lumen 12, and the first ultrasonic transducer 2 and the at least two second ultrasonic transducers 3 can be electrically connected to the control unit through the conductor wire 20, so that the first ultrasonic transducer 2 and the at least two second ultrasonic transducers 3 can be controlled through the control unit to perform ablation. The inflow lumen 13 and the outflow lumen 14 are respectively in communication with a liquid inlet and a liquid outlet of an external water supply apparatus for circulating liquid, so that the tip balloon 4 can be switched between the inflated state and a released state. It may be understood that the catheter body 1 may further be provided with another lumen according to requirements. This is not specifically limited herein.

As shown in FIG. 1, the first ultrasonic transducer 2 is arranged at the distal end of the catheter body 1 and is configured to be electrically connected to the control unit through the conductor wire 20. Specifically, referring to the orientation shown in FIG. 1, the first ultrasonic transducer 2 is arranged parallel to a central axis O of the catheter body 1 and thus under the control of the control unit, converts a pulse electrical signal into a mechanical signal, and generates a single ultrasonic wave to calculate a tissue morphology change and a temperature change by analyzing an echo signal. In other words, the first ultrasonic transducer 2 can work in a measurement mode under the control of the control unit. In this way, a distance to the inner wall of the blood vessel may be measured by using the first ultrasonic transducer 2 to calculate the ablation target area. Moreover, the first ultrasonic transducer 2 can further measure a current temperature of the ablation target area.

In addition, in another embodiment, the first ultrasonic transducer 2 may also be replaced with an infrared measurement apparatus to measure a temperature and a distance through infrared rays. It may be understood that temperature and distance measurement via infrared rays belongs to the related art and is not described in detail herein. A specific type of the infrared measurement apparatus may be adaptively selected according to requirements to satisfy use requirements for different measurement scenarios.

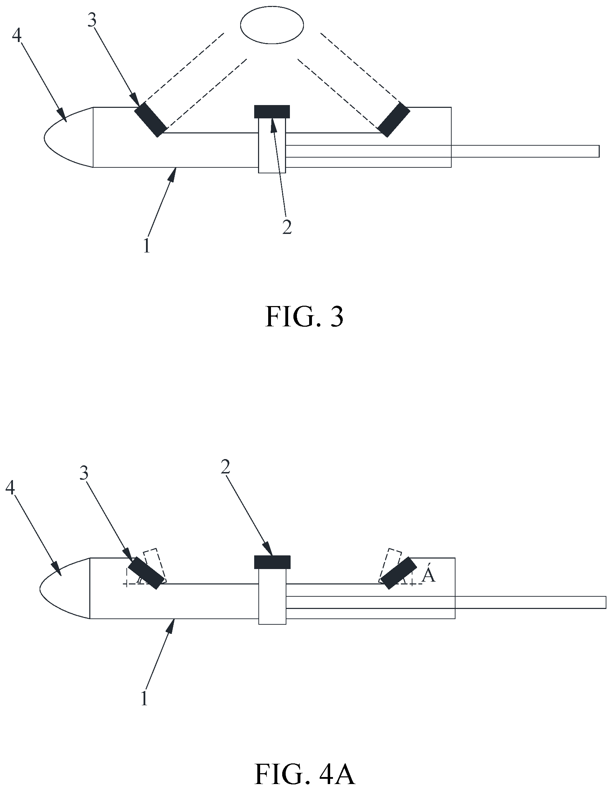

As shown in FIG. 1, the at least two second ultrasonic transducers 3 are arranged at the distal end of the catheter body 1 and are configured to be electrically connected to the control unit through the conductor wire 20. In this embodiment, an example in which two second ultrasonic transducers 3 are arranged is used for description. Specifically, a set included angle α is formed between the second ultrasonic transducers 3 and the central axis O of the catheter body 1, and the two second ultrasonic transducers 3 are mutually symmetrical about the first ultrasonic transducer 2 to determine an ablation focal point and perform focused ablation (referring to FIG. 3). Under the control of the control unit, the second ultrasonic transducers 3 convert electrical signals into mechanical vibration to continuously generate ultrasonic waves and heat an ablation target area in a focus range of energy transferred by acoustic waves. In other words, the second ultrasonic transducers 3 can work in an ablation mode under the control of the control unit. Moreover, in this embodiment, the first ultrasonic transducer 2 and the second ultrasonic transducers 3 are made to correspond to different portions of a blood vessel by rotating the catheter body 1, so as to implement 360° all-around ablation.

It may be understood that, as the second ultrasonic transducers 3 continuously performs ablation, the temperature of the ablation target area gradually increases. In this process, the first ultrasonic transducer 2 continuously detects a current temperature of the ablation target area and feeds back the current temperature to the control unit. Correspondingly, the control unit calculates a current output power of the second ultrasonic transducers 3 based on the current temperature of the ablation target area and based on a preset algorithm. It may be learned that in an actual ablation process, with continuous ablation performed by the second ultrasonic transducers 3, the ablation power of the second ultrasonic transducers 3 is continuously changed, until finally the control unit determines that an ablation endpoint is reached (how to determine the ablation endpoint is described below). Therefore, automated focused ablation may be implemented, thereby improving ablation efficiency. An automated control manner is used to replace a manner of manually determining an ablation state, thereby helping reduce an error rate of manually determining the ablation state, and ensuring consistency of an ablation effect.

As shown in FIG. 1, the tip balloon 4 is arranged at the distal end of the catheter body 1 and is in communication with the inflow lumen 13 and the outflow lumen 14. Therefore, a liquid may be fed into the tip balloon 4 through an external liquid supply apparatus, so that the tip balloon 4 is continuously inflated until abutting against vascular wall. In addition, in this embodiment, preferably, a flexible electrode is mounted on an outer side of the tip balloon 4, and a pressure sensor is mounted on an inner side of the tip balloon 4. The flexible electrode and the pressure sensor are both electrically connected to the control unit to detect impedance and a water pressure. Therefore, in a process in which the tip balloon 4 is continuously inflated, the control unit 1 can determine whether the tip balloon 4 achieves full wall apposition based on a received impedance and water pressure, so as to ensure wall apposition performance of the tip balloon 4. Preferably, the tip balloon 4 is made of a silicon material, and the flexible electrode is a flexible circuit board (FPC).

In addition, it may be understood that the external liquid supply apparatus maintains a current pressure during ablation to continually feed a liquid through the catheter body 1, thereby cooling an interior of the tip balloon 4 while ensuring that the tip balloon 4 remains a required size. After the liquid enters the tip balloon 4 through the inflow lumen 13 of the catheter body 2, the liquid is then discharged from the outflow lumen 14 to a storage apparatus for recycling.

### Second embodiment

As shown in FIG. 4A, the second embodiment of the present invention provides a focused ultrasound ablation catheter including a catheter body 1, a first ultrasonic transducer 2, at least two second ultrasonic transducers 3, and a tip balloon 4. This embodiment differs from the first embodiment in that the second ultrasonic transducers 3 are rotatable.

In this embodiment, the second ultrasonic transducers 3 are rotatably arranged at a distal end of the catheter body 1 to adjust a magnitude of a set included angle α. Specifically, an additional guide wire (not shown in the figure) may be arranged in a conductor wire lumen 12, and the guide wire is connected to the second ultrasonic transducers 3, so that the second ultrasonic transducers 3 are driven to rotate by pulling the guide wire, and the magnitude of the set included angle α can be freely adjusted within a particular angle range (for example, 30° to 60°). It may be understood that after the angle α is changed, focus points and focus regions for the two second ultrasonic transducers 3 are changed accordingly, so that an adaptive adjustment can be performed based on different ablation portions of a blood vessel, thereby improving applicability of a focused ultrasound ablation catheter.

In addition, in this embodiment, the guide wire is arranged to perform a rotational adjustment on the second ultrasonic transducers 3, which is only an implementation. In another embodiment, another adjustment structure may be adaptively used according to requirements to adjust the magnitude of the set included angle α. This is not specifically limited herein.

Other than the above difference, the remaining structure features in this embodiment are the same as those in the first embodiment, and details are not described again.

### Third Embodiment

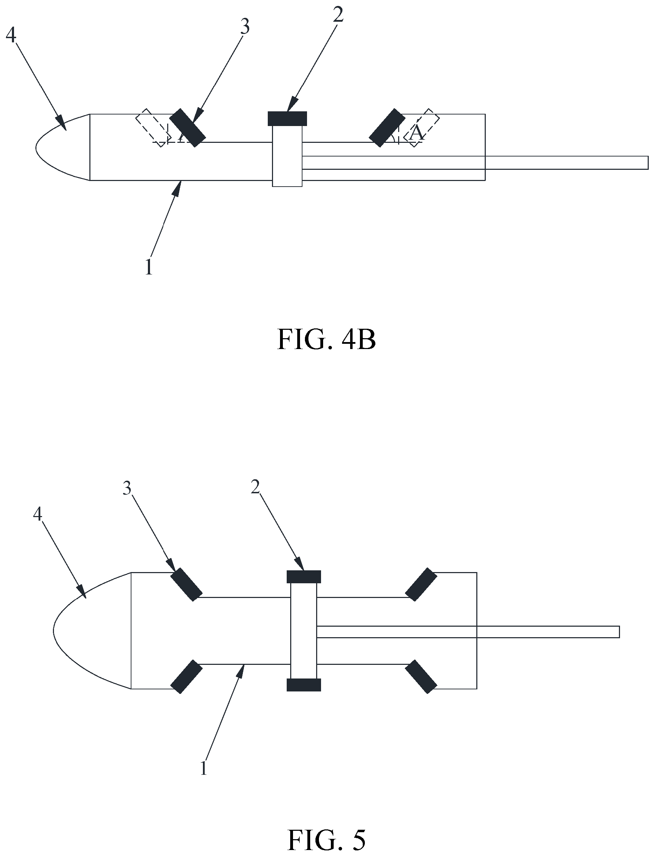

As shown in FIG. 4B, the third embodiment of the present invention provides a focused ultrasound ablation catheter including a catheter body 1, a first ultrasonic transducer 2, at least two second ultrasonic transducers 3, and a tip balloon 4. This embodiment differs from the first embodiment in that the second ultrasonic transducers 3 are movable.

In this embodiment, two second ultrasonic transducers 3 are relatively movable along a central axis of the catheter body 1 with the first ultrasonic transducer 2 as a center for adjusting a relative distance between the two second ultrasonic transducers 3. Specifically, an additional guide wire (not shown in the figure) may be arranged in a conductor wire lumen 12, and the guide wire is connected to the second ultrasonic transducers 3, so that the second ultrasonic transducers 3 are driven to move along the central axis of the catheter body by pulling the guide wire, and the two second ultrasonic transducers 3 are moved closer to each other or farther from each other, thereby adjusting a distance between the two. It may be understood that, after the distance between the two second ultrasonic transducers 3 is changed, a size of an ablation target area determined through the two second ultrasonic transducers is also changed accordingly, so that an adaptive adjustment can be performed based on different ablation portions of a blood vessel, thereby improving applicability of a focused ultrasound ablation catheter.

In addition, in this embodiment, the guide wire is arranged to perform a movement adjustment on the second ultrasonic transducers 3, which is only an implementation. In another embodiment, another adjustment structure may be adaptively used according to requirements to adjust the distance between the two second ultrasonic transducers 3. This is not specifically limited herein.

Other than the above difference, the remaining structure features in this embodiment are the same as those in the first embodiment, and details are not described again.

### Fourth Embodiment

As shown in FIG. 5, the fourth embodiment of the present invention provides a focused ultrasound ablation catheter including a catheter body 1, a plurality of first ultrasonic transducers 2, a plurality of second ultrasonic transducers 3, and a tip balloon 4. This embodiment differs from the first embodiment in that the numbers of the first ultrasonic transducers 2 and the second ultrasonic transducers 3 are different.

Specifically, in this embodiment, the plurality of first ultrasonic transducers 2 are arranged, and the plurality of first ultrasonic transducers 2 are evenly distributed around a peripheral direction of the catheter body 1, so as to define a ring shape. Correspondingly, two second ultrasonic transducers 3 inclined by a set included angle α are symmetrically distributed on two sides of each first ultrasonic transducer 2.

Therefore, one ultrasonic transducer 2 and two corresponding ultrasonic transducers 3 may be considered as an ablation group, and a plurality of ablation groups are formed on the catheter body 1 in a peripheral direction. During specific use, any one or several of the ablation groups may be controlled to enter a working state through a control unit, thereby implementing 360° all-around ablation without rotating the catheter body 1. Not only can the ablation efficiency be improved, but convenience of an ablation operation is also improved.

It may be understood that, in another embodiment, the plurality of second ultrasonic transducers 3 may also be helically distributed along an axial direction of the catheter body 1 to form a spiral structure, so that a plurality of ablation groups can be distributed at intervals along a length direction of the catheter body 1. Therefore, not only can the installation convenience of the ablation group be improved, but the focused ultrasound ablation catheter can also cover a larger ablation region, thereby reducing the number of movements and a travel distance of the catheter body 1 within a blood vessel.

Other than the above difference, the remaining structure features in this embodiment are the same as those in the first embodiment, and details are not described again.

### Fifth Embodiment

As shown in FIG. 6, the fifth embodiment of the present invention provides a focused ultrasound ablation catheter including a catheter body 1, a plurality of first ultrasonic transducers 2, a plurality of second ultrasonic transducers 3, and a tip balloon 4. This embodiment differs from the first embodiment in that an external balloon 5 is further included.

Specifically, in this embodiment, the external balloon 5 is arranged at a distal end of the catheter body 1 to wrap the plurality of first ultrasonic transducers 2, the plurality of second ultrasonic transducers 3, and the tip balloon 4. A water circulation apparatus (not shown in the figure) may be further arranged in the external balloon 5 to cool the first ultrasonic transducers 2 and the second ultrasonic transducers 3 through the water circulation apparatus, thereby ensuring working efficiency of the ultrasonic transducers.

In addition, because the external balloon 5 can wrap the first ultrasonic transducers 2, the second ultrasonic transducers 3, and the tip balloon 4, after the focused ultrasound ablation catheter enters a blood vessel of a human body, vacuum isolation may be formed to prevent the ultrasonic transducers from being polluted, helping improve working efficiency of the ultrasonic transducers. In addition, it may be understood that, after the external balloon 5 is arranged, the tip balloon 4 may be removed, and the external balloon 5 is used to detect impedance and a water pressure.

Other than the above difference, the remaining structure features in this embodiment are the same as those in the first embodiment, and details are not described again.

### Sixth Embodiment

As shown in FIG. 7, the sixth embodiment of the present invention provides a focused ultrasound ablation device including a control unit 100, an operating handle 200, and the focused ultrasound ablation catheter 300 according to any one of the foregoing first embodiment to third embodiment.

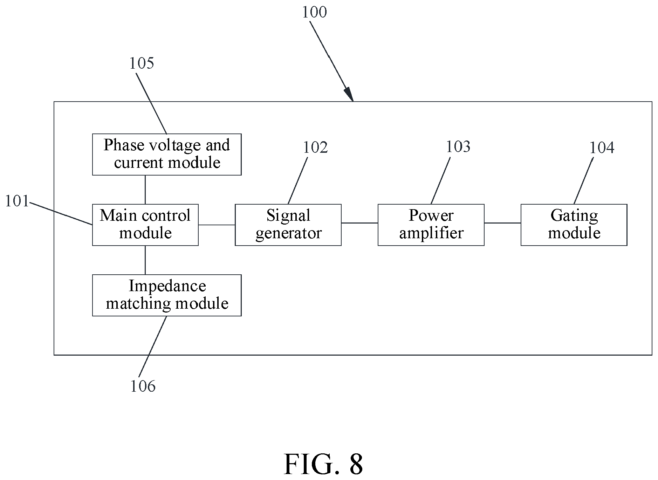

In this embodiment, the control unit 100 is configured for automatic control of ultrasonic ablation. Specifically, as shown in FIG. 8, the control unit 100 includes a main control module 101, a signal generator 102, a power amplifier 103, a gating module 104, a phase voltage and current module 105, and an impedance matching module 106. The main control module 101 is configured to process a signal and data and control automated ablation. The signal generator 102 is connected to the main control module 101 to receive and send an ultrasonic signal. The power amplifier 103 is connected to the signal generator 102 to amplify the ultrasonic signal. The gating module 104 is connected to the power amplifier 103 to select a number of signal channels. The phase voltage and current module 105 is connected to the main control module 101 to detect a parameter and output a matching ultrasonic frequency. The impedance matching module 106 is connected to the main control module 101 and is configured to detect matching impedance and output an ultrasonic signal at a matching power. The operating handle 200 is connected to the control unit 100, so that a doctor performs an operation of an ablation procedure. A proximal end of the focused ultrasound ablation catheter 300 is mounted to the operating handle 200 and is electrically connected to the control unit 100 to perform focused ultrasound ablation under control of the control unit 100.

The control unit 100 continuously adjusts the output power of the second ultrasonic transducers 3 based on a temperature measured by the first ultrasonic transducer 2 until an ablation endpoint is reached. The control unit 100 is further configured to determine whether the ablation endpoint is reached based on determination logic.

In this embodiment, the determination logic specifically includes: when a current temperature of an ablation target area measured by the first ultrasonic transducer 2 reaches a set value and remains stable for a preset duration, and a distance to an inner wall of a blood vessel measured by the first ultrasonic transducer 2 reaches a theoretical value, it is determined that the ablation endpoint is reached.

The following describes specific working steps of the focused ultrasound ablation device in detail.

S1: A device performs self-checking and sends self-checking data to a main control module 101, and visualization is implemented through a display.

S2: A doctor manually starts ablation after the doctor sends the catheter body 1 into a specified tissue of a patient and injects a liquid into the tip balloon 4 by using an external liquid supply apparatus. At the beginning of the liquid injection, a venting program for 3 to 5 seconds is performed. After the venting program is ended, the main control module 101 closes a liquid discharge hole and keeps injecting the liquid through a liquid injection hole.

In this process, a change in a water pressure is detected through a pressure sensor, and a change in impedance is detected through a flexible electrode 5 to transmit the change in a water pressure and the change in impedance to the main control module 101 in real time, so that the main control module 101 determines whether the tip balloon 4 achieves full wall apposition. After the tip balloon 4 is completely adhered to an inner wall of a blood vessel, ultrasonic ablation is started, and the main control module 101 opens the liquid discharge hole and maintains a corresponding water pressure to ensure that a liquid inside the balloon is continuously circulated while the tip balloon 4 maintains a wall apposition state, thereby cooling the tip balloon 4.

S3: After the tip balloon 4 achieves full wall apposition, the main control module 101 sends an ultrasonic signal through the signal generator 102, the signal is amplified through the power amplifier 103, and the phase voltage and current module 105 starts to work to feed back a parameter to the main control module 101 in real time for performing phase detection, and the main control module 101 adjusts a parameter of the signal generator 102 to complete phase matching.

S4: A signal after the phase matching is sent to the first ultrasonic transducer 2 and the second ultrasonic transducers 3 via an appropriate number of channels selected by the gating module 104.

S5: The first ultrasonic transducer 2 starts to measure a distance to the inner wall of the blood vessel and a current temperature of the ablation target area, and transmits temperature and distance data to the main control module 101. Correspondingly, the second ultrasonic transducers 3 release ultrasonic waves of corresponding intensities based on the current output power sent by the main control module 101 to perform focused ablation on the ablation target area.

S6: Data, such as the temperature, the pressure, and the impedance, monitored in real time is fed back to the main control module 101 during the ablation, and the main control module 101 processes the data to determine whether an ablation endpoint is reached based on determination logic and completes an ablation procedure.

Based on the foregoing embodiments, an embodiment of the present invention further provides an ultrasonic ablation system. The system includes the foregoing focused ultrasound ablation device.

Based on the above, the focused ultrasound ablation catheter and device provided in the embodiments of the present invention have the following beneficial effects:
1. The distance to the inner wall of the blood vessel is measured through the first ultrasonic transducers 2 that are horizontally arranged, the ablation target area is calculated, and the current temperature of the ablation target area is measured. In addition, focusing is performed through the second ultrasonic transducers 3 that are obliquely arranged, so that energy transmitted by acoustic waves is focused on a focal ablation region, and other regions are not ablated, thereby effectively protecting vascular intima.
2. The second ultrasonic transducers 3 are rotatable, so that different focal lengths can be implemented through adjustments, and then an adaptability adjustment can be performed based on different ablation positions for a blood vessel to improve applicability of the focused ultrasound ablation catheter.
3. The second ultrasonic transducers are movable, so that a size of a focused ablation region can be implemented through adjustments, and then an adaptability adjustment can be performed based on different ablation positions for a blood vessel to improve the applicability of the focused ultrasound ablation catheter.
4. The plurality of first ultrasonic transducers 2 and the plurality of second ultrasonic transducers 3 may form a plurality of ablation groups. Any one or several of the ablation groups may be controlled to enter a working state through the control unit, thereby implementing all-around ablation within 360° without rotating a catheter body 1. This not only improves ablation efficiency, but also improves convenience of an ablation operation.
5. The focused ultrasound ablation device monitors the water pressure and the impedance during a procedure and for a procedure environment in real time to ensure full wall apposition of the tip balloon 4, thereby improving wall apposition performance and ensuring an ablation effect.
6. The main control module 101 controls an output power of the second ultrasonic transducers 3 to continuously change, thereby implementing automatic ablation, and automatically determines the ablation endpoint during ablation, thereby shortening a procedure time and reducing adverse effects of a patient during a procedure.

It needs to be noted that the foregoing multiple embodiments are merely examples for description. Technical solutions of embodiments may be combined with each other, all of which fall within the protection scope of the present invention.

It should be understood that the orientations or positional relationships indicated by the terms "up", "down", "horizontal", and the like are based on the orientations or positional relationships shown in the accompanying drawings. These terms are merely for ease and brevity of description of the present invention rather than indicating or implying that the apparatuses or elements mentioned need to have specific orientations or need to be constructed or manipulated according to specific orientations, and therefore shall not be construed as any limitations on the present invention.

In addition, terms "first" and "second" are merely used for description, and cannot be understood as indicating or implying relative importance or implying a quantity of indicated technical features. Therefore, features defined by "first" and "second" may explicitly or implicitly include one or more such features. In the descriptions of the present invention, "a plurality of" means two or more, unless otherwise definitely and specifically defined.

The focused ultrasound ablation catheter and device provided in the present invention are described in detail above. For a person of ordinary skill in the art, any apparent change made to the present invention without departing from the essential content of the present invention constitutes infringement of the patent right of the present invention and bears a corresponding legal responsibility.

## Claims

1. A focused ultrasound ablation catheter, comprising:
a catheter body, wherein the catheter body is a multi-lumen tube, and comprises at least a guide wire lumen for passage of a guide wire therethrough and a conductor wire lumen for passage of a conductor wire therethrough;
a first ultrasonic transducer, arranged at a distal end of the catheter body, and configured to be electrically connected to a control unit through the conductor wire, wherein the first ultrasonic transducer is arranged parallel to a central axis of the catheter body and is configured to measure a distance to an inner wall of a blood vessel, to calculate an ablation target area; and the first ultrasonic transducer is further configured to measure a temperature of a target area, which is used for calculating an output power of a second ultrasonic transducer and determining an ablation endpoint; and
at least two second ultrasonic transducers, arranged at the distal end of the catheter body, and configured to be electrically connected to the control unit through the conductor wire, wherein a set included angle is formed between the second ultrasonic transducer and the central axis of the catheter body, and the at least two second ultrasonic transducers are mutually symmetrical about the first ultrasonic transducer to determine an ablation focal point and perform focused ablation.

2. The focused ultrasound ablation catheter according to claim 1, wherein
the second ultrasonic transducer is rotatably arranged at the distal end of the catheter body to adjust a magnitude of the set included angle.

3. The focused ultrasound ablation catheter according to claim 1, wherein
centered on the first ultrasonic transducer, the at least two second ultrasonic transducers are movable relative to each other along the central axis of the catheter body, to adjust a relative distance between the at least two ultrasonic transducers.

4. The focused ultrasound ablation catheter according to claim 1, wherein
a plurality of first ultrasonic transducers are arranged, and the plurality of first ultrasonic transducers define a circular ring along a circumferential direction of the catheter body; and two second ultrasonic transducers that are obliquely arranged are symmetrically distributed on two sides of each of the first ultrasonic transducers.

5. The focused ultrasound ablation catheter according to claim 1, wherein
the second ultrasonic transducer is electrically connected to the controller to receive a current temperature of the ablation target area that is measured by the controller based on the first ultrasonic transducer and calculate a current output power; and
the second ultrasonic transducer emits an ultrasonic wave of a corresponding intensity based on the current output power to perform focused ablation on the ablation target area.

6. The focused ultrasound ablation catheter according to claim 1, further comprising:
a tip balloon, arranged on an end surface of the distal end of the catheter body, in communication with internal lumens of the catheter body, and configured to occlude a blood vessel in an inflated state; and
the internal lumens comprise an inflow lumen for fluid infusion and an outflow lumen for fluid drainage.

7. The focused ultrasound ablation catheter according to claim 6, wherein
a flexible electrode is mounted on an outer side of the tip balloon, a pressure sensor is mounted on an inner side of the tip balloon, the flexible electrode and the pressure sensor are both electrically connected to the control unit to detect impedance and a water pressure.

8. A focused ultrasound ablation device, comprising:
a control unit, configured for automatic control of ultrasonic ablation;
an operating handle, connected to the control unit; and
the focused ultrasound ablation catheter according to any one of claims 1 to 7, wherein a proximal end of the focused ultrasound ablation catheter is mounted to the operating handle and is electrically connected to the control unit; and
the control unit continuously adjusts an output power of the second ultrasonic transducer based on a temperature measured by the first ultrasonic transducer until an ablation endpoint is reached; and the control unit is further configured to determine, based on determination logic, whether the ablation endpoint is reached.

9. The focused ultrasound ablation device according to claim 8, wherein the determination logic specifically comprises:
when a current temperature of an ablation target area measured by the first ultrasonic transducer reaches a set value and remains stable for a preset duration, and a distance to an inner wall of a blood vessel measured by the first ultrasonic transducer reaches a theoretical value, it is determined that the ablation endpoint is reached.

10. The focused ultrasound ablation device according to claim 8, wherein the control unit comprises at least:
a main control module, configured to process a signal and data and control automated ablation;
a signal generator, connected to the main control module, and configured to receive and send an ultrasonic signal;
a power amplifier, connected to the signal generator, and configured to amplify the ultrasonic signal;
a gating module, connected to the power amplifier, and configured to select a number of signal channels;
a phase voltage and current module, connected to the main control module, and configured to detect a parameter and output a matching ultrasonic frequency; and
an impedance matching module, connected to the main control module and configured to detect matching impedance and output an ultrasonic signal at a matching power.
